# EUROPEAN PATENT APPLICATION

(11) **EP 2 957 902 A1**
(43) Date of publication of application: **23.12.2015**
(21) Application number: 14751870.8
(22) Date of filing: 06.02.2014
(51) Int. Cl.: G01N 25/00, G01N 33/22, G01N 5/04

(54) **FUEL-OIL ANALYZER**

(30) Priority: 18.02.2013 JP 2013029175
(71) Applicant: Kawasaki Jukogyo Kabushiki Kaisha, Kobe-shi, Hyogo 650-8670 (JP)
(72) Inventor: OHTANI, Shinichi, Kobe-shi, Hyogo 650-8670 (JP); TOMA, Yoshihiko, Kobe-shi, Hyogo 650-8670 (JP); ENDO, Hiroki, Kobe-shi, Hyogo 650-8670 (JP)
(74) Representative: Leinweber & Zimmermann
(86) International application number: PCT/JP2014/000627
(87) International publication number: WO 2014/125799

(57) **Abstract**

A fuel oil analyzer (1) includes: a sample container (20), in which a sample of a fuel oil is contained; a body case (2) accommodating the sample container (20) therein; a heater (3) configured to heat the sample container (20) in the body case (2); a temperature control unit (9) configured to control a temperature in the body case (2) to be in a predetermined temperature range; temperature sensors (5 to 8) configured to measure a change in the temperature in the body case (2); a weighing scale (15) configured to measure a change in a weight of the fuel oil in the sample container (20); a temperature/weight recording unit (32) configured to record the change in the temperature measured by the temperature sensors (5 to 8) and the change in the weight measured by the weighing scale (15); and an analyzing unit (33) configured to estimate combustion quality of the fuel oil based on the change in the temperature and the change in the weight recorded in the temperature/weight recording unit (32).

## Description

### Technical Field

The present invention relates to fuel oil analyzers suitable for simplified fuel oil analysis.

### Background Art

Conventionally, fuel oils are categorized according to technical specifications. For example, in the case of heavy fuel oils, they are categorized into A-type heavy oils, B-type heavy oils, and C-type heavy oils based on their kinematic viscosity. A-type heavy oils are further subcategorized into No. 1 and No. 2 based on their sulfur content, and C-type heavy oils are further subcategorized into No. 1, No. 2, and No. 3 based on their kinematic viscosity. However, even though the fuel oils are thus subcategorized, the manner in which such a subcategorized fuel oil is refined varies, and also, a mixture in the oil, etc., vary depending on the manufacturer of the oil. For these reasons, the combustion quality of such a subcategorized fuel oil distributed in the market varies.

By taking a ship as one example, while the ship is underway, a fuel oil is loaded into the ship at a port of call. However, the fuel oil may not be suitable for the ship and cause poor combustion performance. There are various troubles arising from such a fuel oil. Examples of the troubles include a combustion trouble where the ignitibility of the fuel oil is poor and continuous combustion of the fuel oil is difficult and a trouble where a mixture in the fuel oil negatively affects the engine.

In the case of, for example, a ship, the kinematic viscosity of a fuel oil usable in the ship is set according to the specifications of the main engine. However, whether or not the fuel oil is suitable for the main engine depends on various factors, such as the type and size of the main engine. Whether or not the fuel oil is suitable for the main engine varies even among main engines of the same specifications due to their individual differences. Therefore, even if there are ships that use a fuel of the same kinematic viscosity, a fuel oil suitable for one of the ships is not necessarily suitable for another one of the ships. That is, even if these ships are supplied with a fuel oil in the same category with the same oil specifications, the fuel oil is not necessarily suitable for all of the ships.

Generally speaking, the combustion quality of a fuel oil is represented by an estimated cetane number (which may hereinafter be abbreviated as "ECN") based on the British Standard (IP541/06). In general, the less the ECN of a fuel oil is, the more the fuel oil is incombustible. It is said that if the ECN is 20 or less, there is a risk of a combustion trouble.

For the above reasons, when a fuel oil is loaded into a ship, a ship crew requests the fuel oil supplier to submit the specifications of the fuel oil, and also, collects a sample of the fuel oil and requests a professional to analyze the sample for quality check.

However, since it takes at least three to five days to obtain the results of the analysis by the professional, ship crews are required to take precautions for preventing the occurrence of troubles, for example, by not using the fuel oil newly loaded in a tank until the analysis results are obtained.

Therefore, there is a high demand for a fuel oil analyzer capable of readily estimating the combustion quality of various fuel oils within a short period of time.

As one example of conventional art intended to meet the demand, there is a quality analyzer intended to determine the ignitibility of a fuel oil by calculating a CCAI value by using the density of the fuel oil at a temperature of 15 °C and the kinematic viscosity of the fuel oil at a temperature of 50 °C (see Patent Literature 1, for example). In addition, the quality analyzer analyzes the proportion of a polymer component contained in the fuel oil, the proportion of insoluble fine particles contained in the fuel oil, and the proportion of sulfur contained in the fuel oil, thereby determining whether or not the fuel oil has suitable quality.

As another example of conventional art, there is a volatile substance content measuring device configured to maintain the internal temperature of the device at a temperature that does not cause combustion of a sample and measure a volatile substance content in the sample based on a change in the weight of the sample (see Patent Literature 2, for example). As yet another example of conventional art, there is an asphalt content measuring device configured to combust an asphalt component in a sample and calculate an asphalt content in the sample based on a decrease in the weight of the sample (see Patent Literature 3, for example).

### Citation List

### Patent Literature

PTL 1: Japanese Laid-Open Patent Application Publication No. 2011-112362
PTL 2: Japanese Laid-Open Patent Application Publication No. 2011-7809
PTL 3: Japanese Laid-Open Patent Application Publication No. 2002-71541

### Summary of Invention

### Technical Problem

However, the invention disclosed in Patent Literature 1 requires a density measuring unit for measuring the density of the fuel oil, and also requires a viscosity measuring unit for measuring the kinematic viscosity of the fuel oil in a state where the fuel oil is heated to 50 °C. This causes the device configuration to be complex and the device size to be large.

Also in the case of intending to determine whether or not a fuel oil has suitable quality by analyzing the proportion of a polymer component contained in the fuel oil, the proportion of insoluble fine particles contained in the fuel oil, and the proportion of sulfur contained in the fuel oil, the configuration of the device for performing these analyses becomes complex. In addition, in order to analyze the proportion of the polymer component contained in the fuel oil, it is necessary to control a temperature for causing a low-molecular component in the fuel oil to combust. Moreover, in order to analyze the proportion of the insoluble fine particles contained in the fuel oil, it is necessary to obtain the insoluble fine particles by filtering a mixture solution to which a solvent such as toluene has been added. Furthermore, in order to analyze the proportion of the sulfur contained in the fuel oil, it is necessary to irradiate the inside of a sample container with X-ray and measure an X-ray dose corresponding to the proportion of the weight of the sulfur. Therefore, in order to realize all of these, the device configuration inevitably becomes complex, and the user of the device is required to be knowledgeable about the handling of the device, the use of which is difficult.

It should be noted that the above-described inventions disclosed in Patent Literatures 2 and 3 are intended to measure a volatile substance content in a sample based on a change in the weight of the sample, or to combust an asphalt component in a sample and calculate an asphalt content in the sample based on a decrease in the weight of the sample. That is, the inventions disclosed in Patent Literatures 2 and 3 are not capable of analyzing the combustion quality of a fuel oil.

In view of the above, an object of the present invention is to provide a fuel oil analyzer capable of readily estimating the combustion quality of a fuel oil within a short period of time with a simple device configuration.

### Solution to Problem

In order to achieve the above-described object, the present invention includes: a sample container, in which a sample of a fuel oil is contained; a body case with an interior space accommodating the sample container; a heating unit configured to heat the sample container in the body case; a temperature control unit configured to control a temperature in the body case to be in a predetermined temperature range; a temperature measuring unit configured to measure a change in the temperature in the body case; a weight measuring unit configured to measure a change in a weight of the fuel oil in the sample container; a recording unit configured to record the change in the temperature measured by the temperature measuring unit and the change in the weight measured by the weight measuring unit; and an analyzing unit configured to estimate combustion quality of the fuel oil based on the change in the temperature and the change in the weight recorded in the recording unit.

According to the above configuration, the fuel oil in the sample container is heated in the body case within the predetermined temperature range. As a result, the weight of the fuel oil decreases steadily, which is recorded in the recording unit. The combustion quality of the fuel oil can be estimated based on a relationship between a change in the weight of the fuel oil and a change in the temperature in the body case. Therefore, in a case where a fuel oil is loaded into a ship, a ship crew can readily estimate the combustion quality of the fuel oil within a short period of time, and can quickly determine whether or not the fuel oil can be suitably used. This allows the ship crew to immediately start using the newly loaded fuel oil, or in some cases, refuse the loading of the fuel oil, without having to wait for analysis results provided from a professional.

The analyzing unit may be configured to: estimate an ignition temperature of the fuel oil based on the change in the temperature and the change in the weight recorded in the recording unit; and estimate an estimated cetane number of the fuel oil based on the estimated ignition temperature.

According to this configuration, the estimated cetane number of the fuel oil can be estimated based on the ignition temperature of the fuel oil, which is estimated based on the change in the weight of the fuel oil and the change in the temperature in the body case, and thereby the combustion quality of the fuel oil can be estimated.

The analyzing unit may be configured to estimate an estimated cetane number of the fuel oil based on a change in the weight of the fuel oil in a predetermined temperature region, the change in the weight being recorded in the recording unit.

According to this configuration, the estimated cetane number of the fuel oil can be estimated based on the change in the weight of the fuel oil in the heated sample container in the predetermined temperature region in the body case, and thereby the combustion quality of the fuel oil can be estimated.

The analyzing unit may be configured to create, based on the change in the temperature and the change in the weight recorded in the recording unit, a weight change curve relative to a heating temperature as a combustion characteristic curve of the fuel oil.

According to this configuration, the combustion characteristic curve of the fuel oil can be obtained based on the change in the weight of the fuel oil and the change in the temperature in the body case, and the combustion quality of the fuel oil can be estimated based on the combustion characteristic curve.

The recording unit may include a storage unit configured to store the combustion characteristic curve.

According to this configuration, the combustion characteristic curve can be stored in the storage unit, and thereby past combustion characteristic curves can be readily referred to when necessary.

The analyzing unit may include a comparing unit configured to compare the created combustion characteristic curve with a past combustion characteristic curve.

According to this configuration, the newly created combustion characteristic curve is compared with the past combustion characteristic curve by the comparing unit, and based on the comparison with the past combustion characteristic curve, it can be estimated whether the fuel oil of the newly created combustion characteristic curve has suitable combustion quality or not.

The analyzing unit may be configured to: cause a display unit to display a plurality of past combustion characteristic curves; and compare the created combustion characteristic curve with the plurality of past combustion characteristic curves.

According to this configuration, the newly created combustion characteristic curve can be compared with the plurality of past combustion characteristic curves, and based on past combustion quality suitability data, it can be estimated whether the combustion quality of the new fuel oil is suitable or not.

The temperature measuring unit may be configured to measure a temperature at a plurality positions around the sample container.

According to this configuration, a change in the temperature in the body case can be measured at the plurality of positions. Therefore, the temperature when a change in the weight of the fuel oil occurs can be measured more precisely.

### Advantageous Effects of Invention

According to the present invention, the combustion quality of a fuel oil can be readily estimated within a short period of time with a simple device configuration. This makes it possible to prevent the occurrence of troubles derived from the fuel oil, and makes it possible to quickly determine, for example, whether or not the loading of the fuel oil should be allowed.

### Brief Description of Drawings

Fig. 1 is a longitudinal sectional view showing a fuel oil analyzer according to one embodiment of the present invention.
Fig. 2 is a block diagram showing functions of a control device of the fuel oil analyzer shown in Fig. 1.
Fig. 3 is a graph showing the results of measurement of temperature changes and weight changes of A-type heavy oils measured by the fuel oil analyzer shown in Fig. 1.
Fig. 4 is a graph showing the results of measurement of temperature changes and weight changes of C-type heavy oils (180 cSt) measured by the fuel oil analyzer shown in Fig. 1.
Fig. 5 is a graph showing the results of measurement of temperature changes and weight changes of C-type heavy oils (380 cSt) measured by the fuel oil analyzer shown in Fig. 1.
Fig. 6 is a graph showing the results of measurement of temperature changes and weight changes of C-type heavy oils (500 cSt) measured by the fuel oil analyzer shown in Fig. 1.
Fig. 7 is a graph showing a relationship between ECNs of fuel oils and their ignition temperatures when ignited in the fuel oil analyzer.
Fig. 8 is a graph showing, for each fuel oil viscosity, a relationship between ECNs of fuel oils and their ignition temperatures, thereby showing a tendency for each fuel oil viscosity.
Fig. 9 is a graph showing a part of the results of the measurements of temperature changes and weight changes shown in Fig. 3 to Fig. 6, such that the part of the results is shown relative to weight changes within predetermined temperature regions.
Fig. 10 is a graph showing a relationship between ECN estimated values and actual ECNs by plotting, thereby showing tendencies, the ECN estimated values each being calculated by using an ECN estimation formula based on values of weight decreases.

### Description of Embodiments

Hereinafter, one embodiment of the present invention is described with reference to the drawings. The embodiment is described below by taking, as one example, a fuel oil analyzer configured to analyze a heavy oil that is a ship fuel oil.

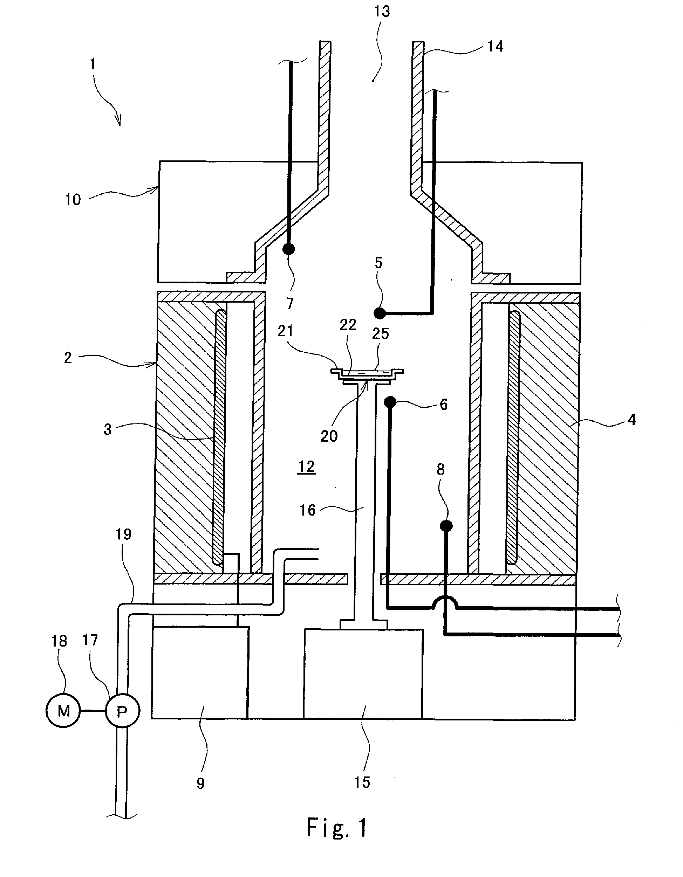

As shown in Fig. 1, a fuel oil analyzer 1 according to the present embodiment includes: a sample container 20, in which a sample of a fuel oil is contained; a body case 2 accommodating the sample container 20 therein; a heater 3 serving as a heating unit heating the sample container 20 in the body case 2; a plurality of temperature sensors 5 to 8 serving as a temperature measuring unit measuring the temperature in the body case 2; a temperature control unit 9 controlling the temperature in the body case 2 within a predetermined temperature range; and a weighing scale 15 serving as a weight measuring unit measuring a change in the weight of the fuel oil in the sample container 20.

The sample container 20 is a dish-shaped container with a central recess 22 formed therein. A flange 21 is formed around the recess 22. The recess 22 has a capacity capable of containing a fuel oil sample 25 in a predetermined amount.

The body case 2 includes an interior space 12, in which the sample container 20 is placed. The body case 2 is a vertical-type case, and the interior space 12 has a cylindrical shape so that the temperature around the sample container 20 increases uniformly.

An openable/closeable cover 10 is provided at the top of the body case 2. Although the cover 10 can be brought into close contact with the top surface of the body case 2, Fig. 1 shows a state where a small gap remains between the cover 10 and the top surface of the body case 2 before the cover 10 is brought into close contact with the top surface.

The sample container 20 is mounted on the top of a support member 16 supported by the weighing scale 15 serving as the weight measuring unit. By setting the weight at the time to "zero", only weight changes of the fuel oil occurring thereafter can be measured by the weighing scale 15.

Further, the central portion of the cover 10 is provided with a tubular part 14 including an exhaust outlet 13, through which the air in the interior space 12 formed in the center of the body case 2 is let out.

The heater 3 is provided around the interior space 12, in which the sample container 20 is placed, such that heat is applied to the interior space 12 from its entire circumference. The heater 3 is embedded in a heat insulating material 4 so that heater wires of the heater 3 will not contact each other. The heater 3 is controlled by the temperature control unit 9 such that the heater 3 is heated a predetermined temperature, and thereby the inside of the body case 2 can be heated.

The plurality of temperature sensors 5 to 8 are provided at upper and lower positions in the interior space 12 of the body case 2. In the present embodiment, temperature changes in the interior space 12 of the body case 2 are measured by the temperature sensors 5 and 7 provided above the sample container 20 and the temperature sensors 6 and 8 provided below the sample container 20. The temperature sensor 5 is provided at a position above the sample container 20, and the temperature sensor 6 is provided at a position below the sample container 20, such that the temperature sensors 5 and 6 are relatively close to each other. The temperature sensor 7 is provided at a position above the sample container 20, and the temperature sensor 8 is provided at a position below the sample container 20, such that the temperature sensors 7 and 8 are remote from each other. Although the temperature sensors 5 to 8 are provided, it will suffice if at least the temperature sensors 5 and 6 positioned above and below the sample container 20 are provided. However, the temperature sensors 7 and 8 are additionally provided in order to measure the temperature in the body case 2 precisely. Thermocouples may be used as the temperature sensors 5 to 8.

The temperature control unit 9 is configured to control the temperature of the heater 3. The temperature control unit 9 controls the heating temperature of the heater 3 to control the temperature in the interior space 12 to be in a predetermined temperature range.

In the present embodiment, the weighing scale 15 has a structure that supports, from below, the support member 16 on which the sample container 20 is mounted. By means of the weighing scale 15, a change, i.e., a decrease, in the weight of the sample container 20 containing the fuel oil sample 25 is measured.

The present embodiment further includes an air pump 17 configured to supply air to the interior space 12 from the outside. The air pump 17 is driven by a motor 18. By means of the air pump 17, air is fed to the interior space 12 through piping 19. The air pump 17 is provided as necessary.

In the present embodiment, the body case 2 is a vertical-type case and the interior space 12 has a cylindrical shape as described above. Accordingly, the air supplied to the interior space 12 by the air pump 17 flows to the exhaust outlet 13 of the cover 10 while generating uniform convection around the sample container 20.

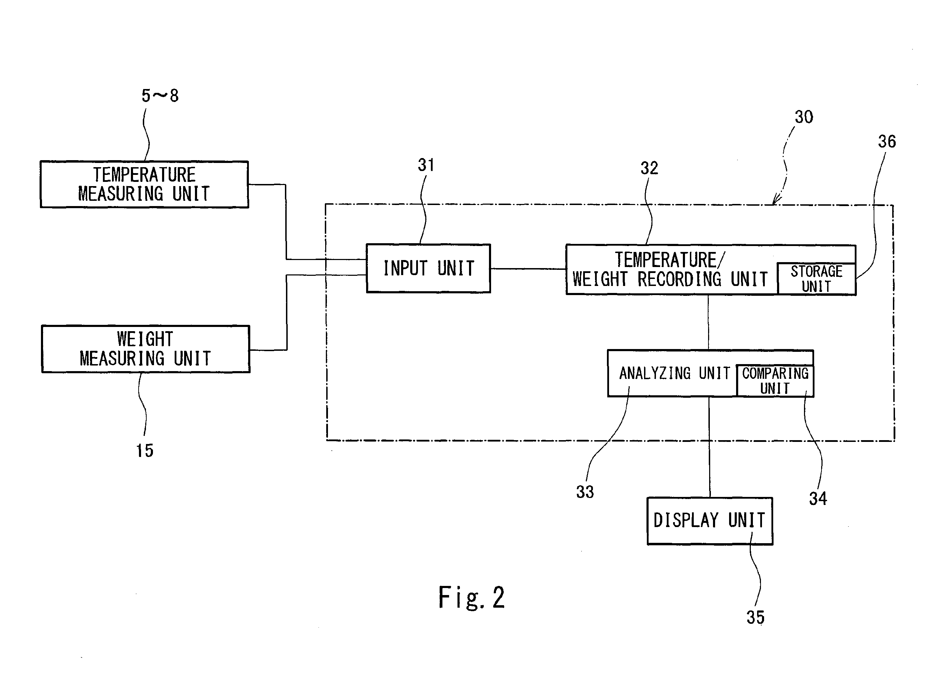

As shown in Fig. 2, the fuel oil analyzer 1 includes a control device 30. The control device 30 includes: a temperature/weight recording unit (a recording unit) 32, in which the temperatures measured by the temperature sensors (the temperature measuring unit) 5 to 8 and the weight measured by the weighing scale (the weight measuring unit) 15 are recorded via an input unit 31; and an analyzing unit 33 configured to estimate the combustion quality of the fuel oil based on temperature changes and weight changes recorded in the temperature/weight recording unit 32.

As described below, the analyzing unit 33 is configured to estimate the ECN of the fuel oil based on temperature changes and weight changes recorded in the temperature/weight recording unit 32, thereby estimating the combustion quality of the fuel oil. The analyzing unit 33 is, for example, a CPU capable of numerical calculation.

The temperature/weight recording unit 32 includes a storage unit 36 storing a combustion characteristic curve. The storage unit 36 stores a created combustion characteristic curve, and allows the stored combustion characteristic curve to be readily referred to as a past combustion characteristic curve. The analyzing unit 33 according to the present embodiment includes a comparing unit 34. As described below, when the combustion characteristic curve of the fuel oil is newly obtained, the obtained combustion characteristic curve can be compared with the past combustion characteristic curve by the comparing unit 34.

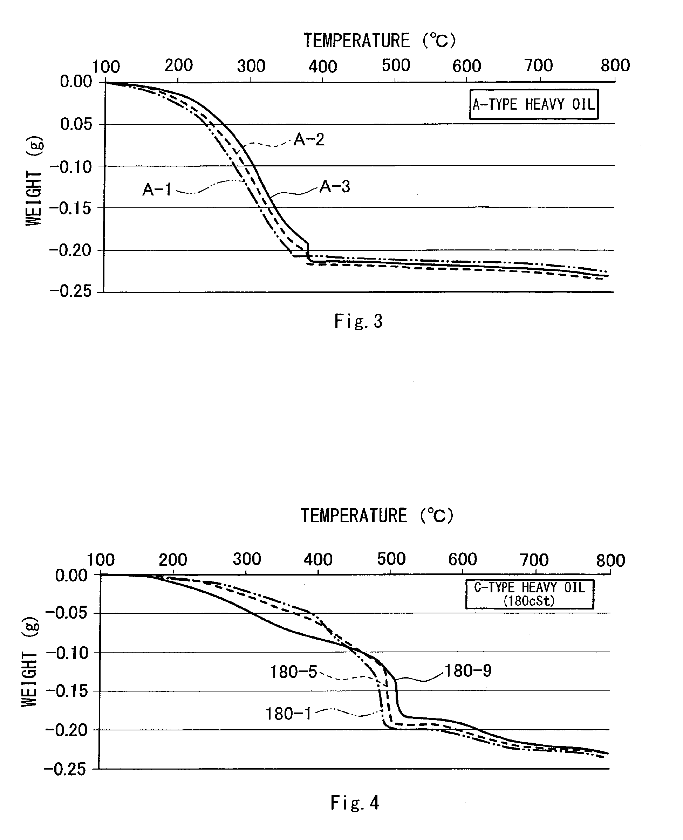

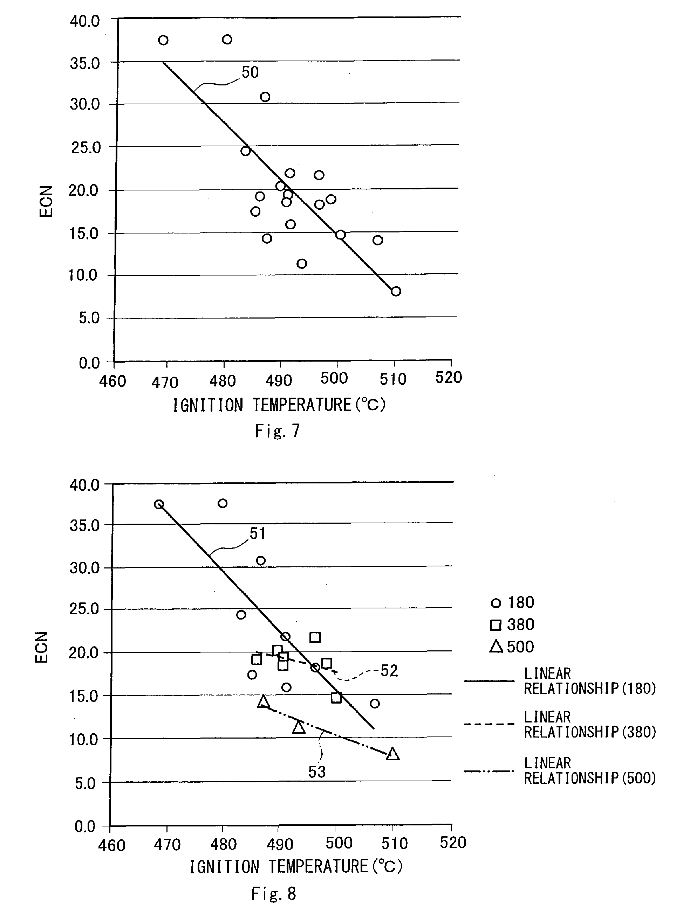

The present embodiment further includes a display unit 35 configured to display the results of analysis by the analyzing unit 33. The display unit 35 is a monitor, for example. The display unit 35 may display, for example, graphs shown in Figs. 7 and 8, ECN estimated values each obtained by using an ECN estimation formula, which will be described below, and combustion characteristic curves as shown in Figs. 3 to 6, which will be described below.

In the present embodiment, the temperature/weight recording unit (the recording unit) 32 is a single unit. However, as an alternative, a temperature recording unit and a weight recording unit may be separately provided. The temperature/weight recording unit 32 and the analyzing unit 33 may be realized by a personal computer, for example. In this case, temperature and weight measurement results may be transmitted to the personal computer, and the personal computer may be configured to perform the analysis and display the results of the analysis.

According to the fuel oil analyzer 1 with the above-described configuration, the combustion quality of the fuel oil can be analyzed in a manner described below.

First, the fuel oil sample 25 in a predetermined amount is put in the sample container 20, and the sample container 20 is mounted on the support member 16 of the weighing scale 15. Then, the cover 10 is closed.

Next, the heater 3 is energized, and thereby the interior space 12 of the body case 2 is heated. The internal temperature of the body case 2 changes as a result of the heating, and the temperature change is measured by the temperature sensors 5 to 8 serving as the temperature measuring unit. Temperatures measured by the temperature sensors 5 to 8 are each recorded in the temperature/weight recording unit 32 via the input unit 31.

When the fuel oil sample 25 in the sample container 20 evaporates or ignites as a result of an increase in the internal temperature of the body case 2, the weight of the fuel oil sample 25 decreases. The decrease in the weight is measured by the weighing scale 15 serving as the weight measuring unit. The measured weight decrease is recorded in the temperature/weight recording unit 32 via the input unit 31.

Then, based on a temperature change obtained by increasing the internal temperature of the body case 2 to a predetermined temperature and a weight change of the fuel oil obtained in the course of the temperature change, the analyzing unit 33 estimates the combustion quality of the fuel oil sample 25 in a manner described below.

First, when the weight of the fuel oil has decreased greatly and the internal temperature of the body case 2 has increased greatly, the analyzing unit 33 estimates that the temperature at the time is the ignition temperature of the fuel oil. Then, the analyzing unit 33 estimates the ECN of the fuel oil based on the ignition temperature. In this manner, the analyzing unit 33 may estimate the combustion quality of the fuel oil. In this manner of estimation, whether the ECN is, for example, higher than 20 or not, may be displayed concurrently during the estimation.

Further, the analyzing unit 33 measures a change in the internal temperature of the body case 2 and an amount of decrease in the weight of the fuel oil over the temperature region of the internal temperature change of the body case 2. Then, based on the amount of decrease in the weight of the fuel oil, the analyzing unit 33 may estimate the ECN of the fuel oil by using an ECN estimation formula described below. In this manner, the analyzing unit 33 may estimate the combustion quality of the fuel oil. Also in this manner of estimation, whether the ECN is, for example, higher than 20 or not, may be displayed concurrently during the estimation.

Still further, based on temperature and weight changes recorded in the temperature/weight recording unit 32, the analyzing unit 33 may create a weight change curve relative to the heating temperature as a combustion characteristic curve of the fuel oil, and may estimate the combustion quality of the fuel oil based on the combustion characteristic curve. The advantage of this manner of estimation is, for example, that the estimation can be made based on, for example, past experiences in which the weight of a fuel oil with high quality decreased steadily at low temperatures whereas the weight of a fuel oil with low quality decreased gently at low temperatures and then decreased rapidly at high temperatures.

In addition, in the estimation of the combustion quality based on the combustion characteristic curve, the combustion characteristic curve of a currently tested fuel oil and the combustion characteristic curve of a fuel oil that was tested in the past and that is in the same category as that of the currently tested fuel oil may be displayed on the display unit 35, and the comparing unit 34 of the analyzing unit 33 may compare these combustion characteristic curves, thereby estimating the combustion quality. In this manner of combustion quality estimation, for example, the combustion characteristic curve of the currently tested fuel oil, the combustion characteristic curve of a fuel oil that was tested in the past and that caused no problem, and the combustion characteristic curve of a fuel oil that was tested in the past and that caused a certain problem are displayed on the display unit 35 at the same time. Then, the combustion characteristic curve of the currently tested fuel oil is compared with these past combustion characteristic curves, and which one of the past combustion characteristic curves is more similar to the combustion characteristic curve of the currently tested fuel oil is determined. Based on the comparison, the combustion quality of the currently tested fuel oil is estimated.

Next, methods of estimating the combustion quality of the fuel oil by the fuel oil analyzer 1 are each described in detail.

First, with reference to Fig. 3 to Fig. 6, graphs showing temperature changes and weight changes of fuel oils measured by the fuel oil analyzer 1 are described. In an example described below, the graphs show combustion characteristic curves, each of which represents a relationship between a temperature change and a weight change. One of the graphs shows combustion characteristic curves regarding three kinds of samples of A-type heavy oils. Each of the other graphs shows, for a different kinematic viscosity, combustion characteristic curves regarding three kinds of samples of C-type heavy oils. In the description below, the samples of A-type heavy oils are denoted by respective numerals 1 to 3, and the samples of C-type heavy oils are denoted by respective numerals and their own kinematic viscosities. It should be noted that each of the graphs in Fig. 3 to Fig. 6 shows combustion characteristic curves of only three kinds of fuel oils so that differences among the curves can be readily seen. However, each of the graphs may show combustion characteristic curves of more kinds of fuel oils.

The graphs in Fig. 3 to Fig. 6 are created based on the results of tests that were performed by the above-described fuel oil analyzer on 26 kinds of fuel oils whose combustion qualities were known in advance. In the tests, three kinds (A-1 to A-3) of A-type heavy oils, nine kinds (180-1 to 180-9) of C-type heavy oils (180 cSt), eight kinds (380-1 to 380-8) of C-type heavy oils (380 cSt), and six kinds (500-1 to 500-6) of C-type heavy oils (500 cSt), i.e., a total of 26 kinds of fuel oils, were tested. The number of kinds of fuel oils to be tested may be further increased, and thereby the estimation precision can be improved.

The ECN of each of the 26 kinds of tested fuel oils was actually measured by a dedicated measuring device, and can be compared with an estimated ECN value obtained in a manner described below. For each of the 26 kinds of tested fuel oils, their ignition temperature and an amount of decrease in their weight due to evaporation or ignition, obtained as a result of performing the tests by the fuel oil analyzer 1, were also actually measured.

Fig. 3 is a graph showing temperature changes and weight changes of A-type heavy oils measured by the fuel oil analyzer 1. As shown in Fig. 3, although a fuel oil of a combustion characteristic curve A-1 shown as a two-dot chain line, a fuel oil of a combustion characteristic curve A-2 shown as a dashed line, and a fuel oil of a combustion characteristic curve A-3 shown as a solid line contain different components from one another, these combustion characteristic curves are substantially the same, and all of the combustion characteristic curves indicate that the weight decreases steadily in accordance with an increase in the temperature to about 390 °C and then the weight gently decreases in accordance with an increase in the temperature.

Fig. 4 is a graph showing temperature changes and weight changes of C-type heavy oils (180 cSt) measured by the fuel oil analyzer 1. As shown in Fig. 4, since a fuel oil of a combustion characteristic curve 180-1 shown as a two-dot chain line and a fuel oil of a combustion characteristic curve 180-5 shown as a dashed line contain different components from each other, these combustion characteristic curves are slightly different from each other, but both of the combustion characteristic curves indicate that: the weight decreases steadily in accordance with an increase in the temperature; then after the temperature exceeds about 400 °C, the weight decreases to a slightly greater degree; then the weight decreases greatly when the temperature reaches about 500 °C; and thereafter, the weight decreases gently in accordance with an increase in the temperature. Meanwhile, in the case of a fuel oil of a combustion characteristic curve 180-9 shown as a solid line, the combustion characteristic curve indicates that: the weight decreases steadily in accordance with an increase in the temperature to about 500 °C; then the weight decreases greatly when the temperature exceeds about 500 °C; and thereafter the weight decreases gently in accordance with an increase in the temperature.

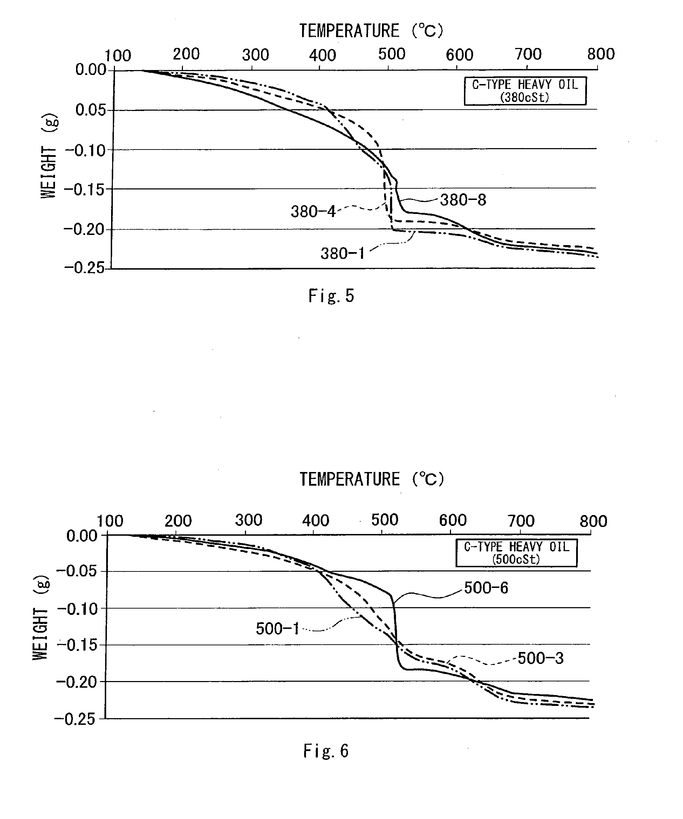

Fig. 5 is a graph showing temperature changes and weight changes of C-type heavy oils (380 cSt) measured by the fuel oil analyzer 1. As shown in Fig. 5, in the case of a fuel oil of a combustion characteristic curve 380-1 shown as a two-dot chain line, the combustion characteristic curve indicates that: the weight decreases steadily in accordance with an increase in the temperature; then after the temperature exceeds about 400 °C, the weight decreases to a slightly greater degree; then the weight decreases greatly when the temperature reaches about 500 °C; and thereafter, the weight decreases gently in accordance with an increase in the temperature. In the case of a fuel oil of a combustion characteristic curve 380-4 shown as a dashed line, the combustion characteristic curve indicates that the weight decreases steadily in accordance with an increase in the temperature to about 500 °C; then the weight decreases greatly when the temperature reaches about 500 °C; and thereafter, the weight decreases gently in accordance with an increase in the temperature. Meanwhile, in the case of a fuel oil of a combustion characteristic curve 380-8 shown as a solid line, the combustion characteristic curve indicates that: in accordance with an increase in the temperature, the weight first decreases more greatly than in the case of the other fuel oils; then after the temperature exceeds about 500 °C, the weight decreases to a greater degree; and thereafter, the weight decreases gently in accordance with an increase in the temperature.

Fig. 6 is a graph showing temperature changes and weight changes of C-type heavy oils (500 cSt) measured by the fuel oil analyzer 1. As shown in Fig. 6, in the case of a fuel oil of a combustion characteristic curve 500-1 shown as a two-dot chain line, the combustion characteristic curve indicates that: the weight decreases steadily in accordance with an increase in the temperature; then after the temperature exceeds about 400 °C, the weight decreases to a slightly greater degree; then after the temperature exceeds about 500 °C, the weight decreases gently in accordance with an increase in the temperature. In the case of a fuel oil of a combustion characteristic curve 500-3 shown as a dashed line, the combustion characteristic curve indicates that: the weight decreases steadily in accordance with an increase in the temperature to about 500 °C; and then after the temperature exceeds about 500 °C, the weight decreases gently in accordance with an increase in the temperature. Meanwhile, in the case of a fuel oil of a combustion characteristic curve 500-6 shown as a solid line, the combustion characteristic curve indicates that: the weight decreases gently until the temperature exceeds about 500 °C; then after the temperature exceeds about 500 °C, the weight decreases greatly; and thereafter the weight decreases gently in accordance with an increase in the temperature.

As described above, as shown in Fig. 3 to Fig. 6, according to the fuel oil analyzer 1, even if there are fuel oils subcategorized in the same category, if the fuel oils are supplied from different sources, respectively, then combustion characteristic curves each indicating a different temperature change-weight change relationship can be obtained from the fuel oils.

Each combustion characteristic curve represents changes in the weight of a sample oil, the changes occurring in accordance with temperature increase. The combustion characteristic curve of each A-type heavy oil indicates characteristics that are clearly different from characteristics indicated by the combustion characteristic curve of each C-type heavy oil.

In the case of the A-type heavy oils, each sample oil entirely evaporates before the temperature reaches 400 °C. On the other hand, in the case of the C-type heavy oils, the combustion characteristic curve of a fuel with a relatively large ECN value, i.e., a fuel with high combustion quality, is different from the combustion characteristic curve of a fuel with a relatively small ECN value, i.e., a fuel with low combustion quality. The higher the combustion quality of a fuel, the more the fuel tends to start evaporating showing a steep slope from a temperature of about 400 °C and continue evaporating or ignite in the temperature range of about 480 °C to 520 °C. In the case of igniting, the weight of the sample oil decreases rapidly due to the generated heat.

In the case of the C-type heavy oils of 180 cSt and 380 cSt, a sample oil with low combustion quality (indicated by a solid line) starts evaporating noticeably from a very early stage of about 300 °C, and ignites in a state where the slope of the evaporation curve is gentle. On the other hand, the evaporation curve of a sample oil with high combustion quality is such that, at a temperature of about 400 °C, the slope of the evaporation curve suddenly changes from a gentle slope into a steep slope and the sample oil ignites.

In the case of the C-type heavy oils of 500 cSt, the slope of the evaporation curve of a sample oil with low combustion quality (indicated by a solid line) is gentle overall, and the slope of the evaporation curve of a sample oil with high combustion quality (i.e., a sample oil that does not ignite) becomes steep from a temperature of about 400 °C.

Based on the above-described analyses and findings, the combustion quality of a fuel oil to be used (i.e., analysis subject) can be estimated from its combustion characteristic curve.

The fuel oil combustion characteristic curve obtained in the above-described manner can be readily stored, for example, in the form of a database in the storage unit 36 of the control device 30 together with information such as "port of call", "name", "supplier", etc. By storing the combustion characteristic curve in the form of a database, the stored combustion characteristic curve can be later utilized as a sample to be compared with the combustion characteristic curve of another fuel oil to estimate whether the quality of this other fuel oil is high or not. The stored combustion characteristic curve can be utilized in the following manner: the combustion characteristic curve of a fuel oil that is in the same category as that of a currently tested fuel oil is selectively obtained from the past database as a combustion characteristic curve to be displayed on the display unit 35, and the displayed combustion characteristic curve is compared with the combustion characteristic curve of the currently tested fuel oil; and whether or not the currently tested fuel oil has suitable combustion quality can be estimated based on the comparison. For example, the display unit 35 displays the combustion characteristic curve of a fuel oil that caused a problem in the past and the combustion characteristic curve of a fuel oil that caused no problem in the past. Then, which one of these combustion characteristic curves from the past is more similar to the combustion characteristic curve of the currently tested fuel oil is confirmed. Accordingly, the combustion quality of the currently tested fuel oil can be estimated in the following manner: if the combustion characteristic curve of the fuel oil that caused a problem in the past is more similar to the combustion characteristic curve of the currently tested fuel oil, then it can be estimated that the currently tested fuel oil has a problem; and if the combustion characteristic curve of the fuel oil that caused no problem in the past is more similar to the combustion characteristic curve of the currently tested fuel oil, then it can be estimated that the currently tested fuel oil has no problem.

Fig. 7 is a graph showing a relationship between ignition temperatures obtained from the results of the tests performed by the fuel oil analyzer 1 and ECNs. From this graph, the relationship between the ignition temperatures and the ECNs can be estimated as a linear relationship 50 represented by a solid line.

Accordingly, if the relationship as shown in Fig. 7 is derived in advance, then in this example, the sample 25 of a C-type heavy oil is heated by the fuel oil analyzer 1; an ignition temperature is estimated based on changes in the temperature and weight of the sample 25; and the ECN of the fuel oil can be readily estimated from the ignition temperature based on the relationship in Fig. 7. The estimation of the ECN is performed by the above-described analyzing unit 33 shown in Fig. 2, and can be displayed on the display unit 35.

In the graph shown in Fig. 8, the data of the above-described relationship between the ignition temperatures and the ECNs shown in Fig. 7 is organized for each kinematic viscosity of C-type heavy oil. The graph in Fig. 8 shows plotting results for each kinematic viscosity of C-type heavy oil. The C-type heavy oils (180 cSt) are each plotted as a circle, and a linear relationship thereof is represented by a solid line 51. The C-type heavy oils (380 cSt) are each plotted as a square, and a linear relationship thereof is represented by a dashed line 52. The C-type heavy oils (500 cSt) are each plotted as a triangle, and a linear relationship thereof is represented by a two-dot chain line 53.

Since the data is organized for each kinematic viscosity of C-type heavy oil in the above-described manner, in the case of heating the sample 25 of a C-type heavy oil by the fuel oil analyzer 1 and estimating an ignition temperature based on changes in the temperature and weight of the sample 25, the ECN of the fuel oil can be estimated more precisely from the ignition temperature based on a relationship in Fig. 8, the relationship corresponding to the kinematic viscosity of the fuel oil. The estimation of the ECN is performed by the above-described analyzing unit 33 shown in Fig. 2, and can be displayed on the display unit 35.

The above-described manner of estimation is for estimating the ECN of a fuel oil based on its ignition temperature in a case where the fuel oil has ignited while being tested. However, some fuel oils may only evaporate without igniting.

Described next is a fuel oil analyzer applicable to a case where the weight of a fuel oil has changed only through evaporation without igniting, the fuel oil analyzer being configured to estimate the ECN of the fuel oil by utilizing evaporation characteristics. Although it is considered that the combustion quality of a fuel oil can be obtained from evaporation characteristics specific to the fuel oil, there are cases where the fuel oil ignites, and also, there are cases where the fuel oil does not ignite. The fuel oil analyzer described below is usable in cases where the fuel oil does not ignite.

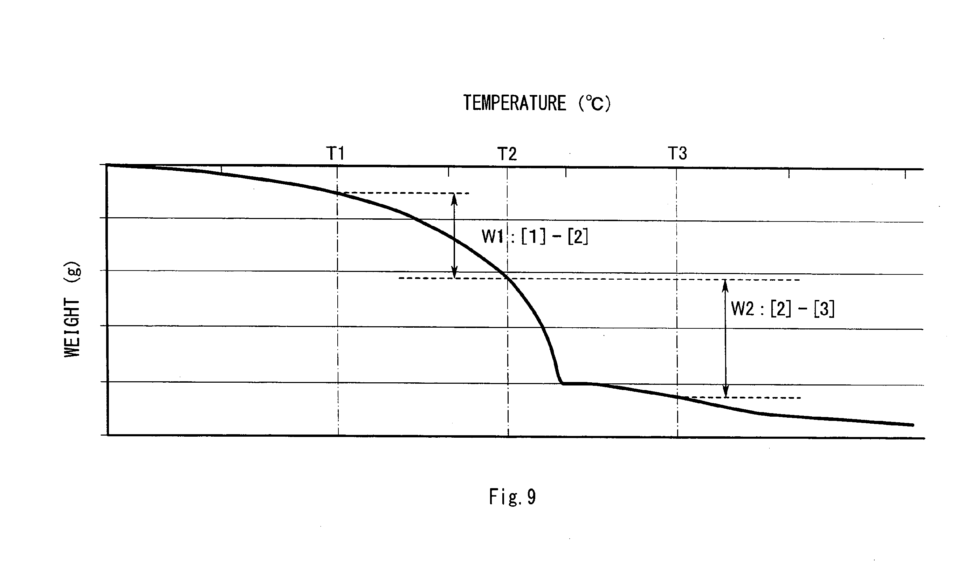

Fig. 9 shows predetermined temperature regions for defining combustion quality parameters from changes in the weight of a fuel oil, the predetermined temperature regions being set in order to obtain evaporation characteristics of the fuel oil. A low-temperature region of T1 to T2 and a high-temperature region of T2 to T3 are set as the predetermined temperature regions. The predetermined temperature regions are set corresponding to great weight changes of the fuel oil, the great weight changes occurring due to evaporation or ignition. An amount of decrease in the weight in the low-temperature region is W1, and an amount of decrease in the weight in the high-temperature region is W2.

In the case of adopting these settings, even if the fuel oil ignites, it is considered that, at the temperature T3, the weight curve after the ignition substantially coincides with the weight curve in a case where the fuel oil only evaporates. Therefore, in this case, the influence of the ignition at the temperature T3 can be ignored. W1 indicates an evaporation amount before main combustion of the fuel occurs. This evaporation exerts a priming effect, and generally speaking, the greater the value of the evaporation amount, the higher the combustion quality. W2 corresponds to a decrease in the fuel due to its main combustion.

Then, since all of the ECNs of the above-described C-type heavy oils have been obtained as actual measurement values, multiple regression analysis using W1 and W2 as parameters is performed for each fuel oil kinematic viscosity based on the ECN actual measurement values, and thereby an ECN estimation formula can be created for each kinematic viscosity.

In order to improve the precision of the ECN estimation, the multiple regression analysis is performed for each fuel oil kinematic viscosity, and an ECN estimation formula is created for each kinematic viscosity. For example, the ECN estimation formula is in the following form: "ECN (estimated value) = A + B × W1 + C × W2". Here, A, B, and C represent coefficients calculated by the multiple regression analysis.

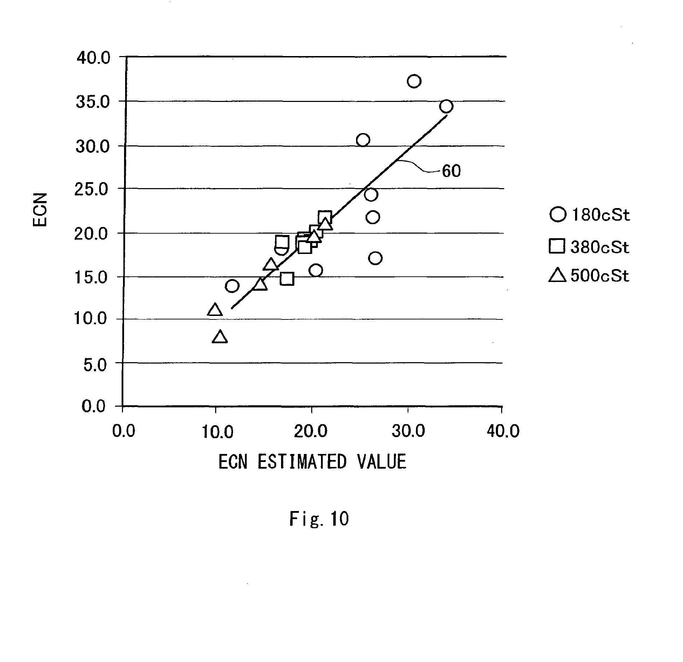

Fig. 10 shows, for each kinematic viscosity, a relationship between ECN actual measurement values and ECN estimated values obtained by the above ECN estimation formula. It can be considered from Fig. 10 that there is a linear relationship 60 between the ECN actual measurement values and the ECN estimated values. Correlation coefficients in the case of performing the ECN estimation with this method were calculated, and it was found that the estimation precision in this case was substantially the same as in the case of performing the ECN estimation based on an ignition temperature.

Thus, according to the fuel oil analyzer 1, by measuring an amount of decrease in the weight of the fuel oil in each predetermined temperature region and performing calculation based on the measured amount of decrease in the weight by using the ECN estimation formula, the ECN of the sample of the fuel oil can be estimated.

As described above, according to the fuel oil analyzer 1, a predetermined amount of fuel oil is put in the sample container 20; the sample container 20 is heated to a predetermined temperature in the interior space 12 of the body case 2; a change in the temperature of the interior space 12 and a change, i.e., a decrease, in the weight of the fuel oil obtained in the course of the temperature change are measured; the results of the measurement are analyzed; and based on the analysis, a combustion characteristic curve is created. The created combustion characteristic curve is compared with the combustion characteristic curve of a fuel whose combustion quality has been evaluated in advance, and thereby the combustion quality of the fuel oil can be estimated.

Further, by estimating the ECN of the fuel oil based on its ignition temperature, the combustion quality of the fuel oil can be readily estimated based on the estimated ECN.

Still further, by estimating the ECN of the fuel oil based on the amount of decrease in the weight of the fuel oil when the fuel oil is heated, the combustion quality of the fuel oil can be readily estimated based on the estimated ECN.

Although the combustion quality of the fuel oil can be readily estimated within a short period of time in either manner, the estimation precision can be further improved by combining these manners of estimation.

Therefore, a ship crew or the like is no longer required to send a fuel oil sample to a professional to ask the professional to analyze the sample and wait for the results of the analysis, and can immediately start using the newly loaded fuel oil, or in some cases, can refuse the loading of the fuel oil.

It should be noted that the fuel oil analyzer 1 according to the above-described embodiment is merely one example. As an alternative, the fuel oil analyzer 1 may be configured such that the body case 2 is provided with the analyzing unit (e.g., a personal computer) 33. Thus, the configuration of the fuel oil analyzer 1 is not limited to the one described in the above embodiment.

In the above-described embodiment, mainly C-type heavy oils are taken as examples of the fuel oil. However, any fuel oil whose combustion quality needs to be estimated can be analyzed in the same manner, and the fuel oil is not limited to C-type heavy oils.

The above embodiment describes non-limiting examples. Various modifications can be made to the embodiment without departing from the spirit of the present invention.

Thus, the present invention is not limited to the above-described embodiment.

### Industrial Applicability

The fuel oil analyzer according to the present invention can be used as a simplified fuel oil analyzer capable of readily analyzing a fuel oil such as a heavy oil within a short period of time as to whether the fuel oil has suitable combustion quality or not.

### Reference Signs List

- 1: fuel oil analyzer
- 2: body case
- 3: heater (heating unit)
- 4: heat insulating material
- 5: temperature sensor (temperature measuring unit)
- 6: temperature sensor (temperature measuring unit)
- 7: temperature sensor (temperature measuring unit)
- 8: temperature sensor (temperature measuring unit)
- 9: temperature control unit
- 10: cover
- 12: interior space
- 13: exhaust outlet
- 14: tubular part
- 15: weighing scale (weight measuring unit)
- 16: supporting member
- 17: air pump
- 18: motor
- 19: piping
- 20: sample container
- 21: flange
- 22: recess
- 25: fuel oil sample
- 30: control device
- 31: input unit
- 32: temperature/weight control unit
- 33: analyzing unit
- 34: comparing unit
- 35: display unit
- 36: storage unit
- 50: linear relationship
- 51: linear relationship
- 52: linear relationship
- 53: linear relationship
- 60: linear relationship
- A-1 to A-3: combustion characteristic curve
- 180-1: combustion characteristic curve
- 180-5: combustion characteristic curve
- 180-9: combustion characteristic curve
- 380-1: combustion characteristic curve
- 380-4: combustion characteristic curve
- 380-8: combustion characteristic curve
- 500-1: combustion characteristic curve
- 500-3: combustion characteristic curve
- 500-6: combustion characteristic curve

## Claims

1. A fuel oil analyzer comprising:
a sample container, in which a sample of a fuel oil is contained;
a body case with an interior space accommodating the sample container;
a heating unit configured to heat the sample container in the body case;
a temperature control unit configured to control a temperature in the body case to be in a predetermined temperature range;
a temperature measuring unit configured to measure a change in the temperature in the body case;
a weight measuring unit configured to measure a change in a weight of the fuel oil in the sample container;
a recording unit configured to record the change in the temperature measured by the temperature measuring unit and the change in the weight measured by the weight measuring unit; and
an analyzing unit configured to estimate combustion quality of the fuel oil based on the change in the temperature and the change in the weight recorded in the recording unit.

2. The fuel oil analyzer according to claim 1, wherein
the analyzing unit is configured to:
estimate an ignition temperature of the fuel oil based on the change in the temperature and the change in the weight recorded in the recording unit; and
estimate an estimated cetane number of the fuel oil based on the estimated ignition temperature.

3. The fuel oil analyzer according to claim 1, wherein
the analyzing unit is configured to estimate an estimated cetane number of the fuel oil based on a change in the weight of the fuel oil in a predetermined temperature region, the change in the weight being recorded in the recording unit.

4. The fuel oil analyzer according to claim 1, wherein
the analyzing unit is configured to create, based on the change in the temperature and the change in the weight recorded in the recording unit, a weight change curve relative to a heating temperature as a combustion characteristic curve of the fuel oil.

5. The fuel oil analyzer according to claim 4, wherein
the recording unit includes a storage unit configured to store the combustion characteristic curve.

6. The fuel oil analyzer according to claim 5, wherein
the analyzing unit includes a comparing unit configured to compare the created combustion characteristic curve with a past combustion characteristic curve.

7. The fuel oil analyzer according to claim 5 or 6, wherein
the analyzing unit is configured to:
cause a display unit to display a plurality of past combustion characteristic curves; and
compare the created combustion characteristic curve with the plurality of past combustion characteristic curves.

8. The fuel oil analyzer according to any one of claims 1 to 7, wherein
the temperature measuring unit is configured to measure a temperature at a plurality positions around the sample container.
